Europäisches Patentamt

European Patent Office

Office européen des brevets

(11) Publication number: 0 344 118
A2

# EUROPEAN PATENT APPLICATION

(21) Application number: 89810373.4

(22) Date of filing: 19.05.89

(51) Int. Cl.⁴: **C 08 L 89/06**
C 08 L 101/00, B 29 C 45/00,
C 09 H 9/02

(30) Priority: 26.05.88 GB 8812502

(43) Date of publication of application:
29.11.89 Bulletin 89/48

(84) Designated Contracting States:
AT BE CH DE ES FR GB GR IT LI LU NL SE

(71) Applicant: **WARNER-LAMBERT COMPANY**
**201 Tabor Road**
**Morris Plains New Jersey 07950 (US)**

(72) Inventor: **Silbiger, Jakob, Dr.**
**Oberwilerstrasse 72**
**CH-4054 Basel (CH)**

**Stepto, Robert Frederick Thomas, Dr.**
**20 Gloucester Road**
**Poynton Cheshire SK12 1JJ (GB)**

(74) Representative: **Silbiger, Jakob, Dr.**
**c/o CAPSUGEL AG Münchensteinerstrasse 41**
**CH-4002 Basel (CH)**

(54) New polymer composition.

(57) A polymer composition as obtained from a melt comprising at least one water-containing destructurized hydrophilic polymer and at least one synthetic essentially water-insoluble thermoplastic polymer and shaped articles made therefrom.

EP 0 344 118 A2

**Description**

**New Polymer Composition**

The present invention refers to a novel polymer composition comprising at least one hydrophilic polymer and at least one synthetic thermoplastic polymer.

It is known that many hydrophilic polymers, which in the dry state, i.e. in the absence of water cannot be melted and decompose at elevated temperature, such as gelatin, form a thermoplastic melt in the presence of a defined amount of water. It is also known that hydrophilic polymers that do melt in the dry state at elevated temperature form thermoplastic melts at those temperatures also in the presence of defined amounts of water.

These hydrophilic polymers containing a defined amount of water can be treated at an elevated temperature and in a closed vessel, thereby at elevated pressure, to form a melt. The process is conveniently carried out in an injection moulding machine or extruder. The starch is fed through the hopper onto a rotating, reciprocating screw. The feed material moves along the screw towards the tip. During this process, its temperature is increased by means of external heaters around the outside of the barrel and by the shearing action of the screw. Starting in the feed zone and continuing in the compression zone, the particulate feed becomes gradually molten. It is then conveyed through the metering zone, where homogenization of the melt occurs, to the end of the screw. The molten material at the tip can then be further treated by injection moulding or extrusion or any other known technique to treat thermoplastic melts, to obtain shaped articles.

This treatment, which is described in the European Patent Application No. 83 301 643.9 (Publication No. 0 090 600) yields an essentially destructurized polymer. The reason for this being that the hydrophilic polymer is heated above the melting and glass transition temperatures of its components so that they undergo endothermic transitions. As a consequence a melting and disordering of the molecular structure takes place, so that an essentially destructurized material is obtained. The expression "hydrophilic polymer" defines the material obtained by such thermoplastic melt formation.

The preferred hydrophilic polymer of the present invention are natural hydrophilic polymers and especially gelatin. Such gelatin is made preferably from acid or alkaline processed ossein , acid processed pigskin, or alkaline processed cattle hide. Said types of various gelatin have a molar mass range of 10,000 to 2,000,000 grammes per mole (g mol$^{-1}$) or a molar mass range of 10,000 to 2,000,000 and 10,000,000 to 20,000,000 g mol$^{-1}$. Such gelatins are known. However, other hydrophilic polymers as described herein are included in this invention. Hydrophilic polymers are polymers with molar masses from approximately $10^3$ to $10^7$ g mol$^{-1}$ carrying functional groups in their backbone and/or in their side-chains which are capable of participating in hydrogen bonds. Such hydrophilic polymers exhibit in their water adsorption isotherm in the temperature range between approximately 0 to 200°C an inflection point close to the water activity point at 0.5.

The obtained articles from such melts are useful materials for many applications. An important property is their biodegradability. In humid air, however, such shaped articles take up water from the air thereby increasing their own moisture content. In consequence such articles made from such hydrophilic materials lose their form stability very quickly, which is a serious disadvantage for many applications.

It is known that synthetic oil-based thermoplastic materials which are hydrophobic and essentially water-insoluble must be processed in the absence of water or volatile materials. It was therefore assumed that water-containing hydrophilic polymers, such as gelatin, could not be used as a thermoplastic component together with hydrophobic essentially water-insoluble polymeric materials such as water-insoluble polyolefines not only due to the mentioned factors but also due to the general incompatible nature of the two types of materials.

It has now been surprisingly found that hydrophilic polymers as mentioned above, containing a defined amount of water, when heated in a closed vessel as described above to form a melt, that such melts made from water-containing hydrophilic polymers are compatible with melts formed by essentially anhydrous thermoplastic synthetic polymers and that the two types or molten materials show an interesting combination of their properties, especially after the melt has solidified.

One very important aspect is the surprisingly improved dimensional stability of such shaped articles made from such hydrophilic polymers blended with such a thermoplastic synthetic material. For example, the blending of destructurized starch with only 2% by weight of a thermoplastic synthetic polymer as described later on, may reduce the shrinkage to about 50% after two days, compared with the shrinkage of a non-blended material.

The present invention refers to a polymer composition as obtained from a melt comprising at least one water-containing destructurized hydrophilic polymer and at least one synthetic essentially water-insoluble thermoplastic polymer.

The present invention refers to said polymer composition in the molten or in the solid form.

The present invention further refers to shaped articles made from said polymer composition.

The present invention further refers to the use of an essentially water-insoluble hydrophilic thermoplastic polymer as a thermoplastic component in the thermoplastic processing of a water-containing hydrophilic polymer.

The present invention further refers to a method of producing said polymer composition in the molten or solid form as well as a method of producing shaped articles from said polymer composition.

Specifically the present invention also refers to a method of producing a polymer composition comprising at least one modified destructurized hydrophilic polymer by heating a hydrophilic polymer having a water content of 5 to 25 % by weight based on the total amount of hydrophilic polymer/water component in a closed volume to elevated temperatures thereby at elevated pressures for a time long enough to form a melt, characterized in that said hydrophilic polymer/water material is mixed with at least one synthetic thermoplastic polymer before melting is initiated.

The term "hydrophilic polymer" is defined above. It includes water-soluble and water-swellable polymers. As such it includes animal gelatin, vegetable gelatins resp. proteins such as sunflower protein, soybean proteins, cotton seed proteins, peanut proteins, rape seed proteins, acrylated proteins; water-soluble polysaccharides, alkylcelluloses hydroxyalkylcelluloses and hydroxyalkylalkylcelluloses, such as: methylcellulose, hydroxy-methylcellulose, hydroxyethylcellulose, hydroxypropylcellulose, hydroxyethylmethylcellulose, hydroxpro-pylmethylcellulose, hydroxybutylmethylcellulose, cellulose esters and hydroxyalkylcellulose esters such as: cellulose acetylphtalate (CAP), Hydroxypropylmethylcellulose (HPMCP); carboxyalkylcelluloses, carboxyalkyl-alkylcelluloses, carboxyalkylcellulose, analogous known polymers made from starch; esters such as: carboxymethylcellulose and their alkalimetal salts; water-soluble synthetic polymers such as: polyacrylic acids and polyacrylic acid esters, polymethacrylic acids and polymethacrylic acid esters, polyvinylalcohols, polyvinylacetatephthalates (PVAP), polyvinylpyrrolidone, polycrotonic acids; suitable are also phthalated gelatin, gelatin succinate, crosslinked gelatin, shellac, cationically modified acrylates and methacrylates possessing, for example, a tertiary or quaternary amino group, such as the diethylaminoethyl group, which may be quaternized if desired; and other similar polymers.

Preferred are natural hydrophilic polymers, especially gelatin.

Examples of essentially water-insoluble thermoplastic materials are polyolefines, such as polyethylene (PE), polyisobutylenes, polypropylenes, vinylpolymers such as poly(vinyl chloride) (PVC), poly(vinyl acetates), polystyrenes; polyacrylonitriles (PAN); polyvinylcarbazols (PVK); essentially water-insoluble poly(acrylic acid) esters or poly(methacrylic acid) esters; polyacetals (POM); polycondensates such as polyamides (PA), thermoplastic polyesters, polycarbonates, poly(alkylene terephthalates); polyarylethers; thermoplastic polyimides; but also poly(hydroxy butyrate) (PHB) and high molar-mass, essentially water-insoluble poly(alkylene oxides) such as polymers of ethylene oxide and propylene oxide as well as their copolymers are included.

Further included are essentially water-insoluble thermoplastic copolymers of the different kinds known such as ethylene/vinyl acetate-copolymers (EVA); ethylene/vinyl alcohol-copolymers (EVAL); ethylene/acrylic acid-copolymers (EAA); ethylene/ethyl acrylate-copolymers (EEA); ethylene/methyl acrylate-copolymers (EMA); ABS - copolymers; styrene/acrylonitrile-copolymers (SAN); as well as their mixtures.

Preferred from these are those with a set processing temperature preferably within the range of 95°C to 210°C, preferably within the range of 95°C to 190°C.

Preferred from these are further those polymers containing polar groups such as ether-, acid or ester groups. Such polymers include e.g. copolymers of ethylene, propylene or isobutylene such as ethylene/vinyl acetate-copolymers (EVA), ethylene/vinyl alcohol-copolymers, ethylene/acrylic acid-copolymers (EAA), ethylene/ ethyl acrylate-copolymers (EEA), ethylene/methacrylate-copolymers (EMA), styrene/acrylonitrile-copolymers (SAN); polyacetals (POM) and their mixtures as mentioned above.

Preferably the solubility parameters of the hydrophilic polymers on the one hand and the hydrophobic polymers on the other hand have a difference in their values of at least 1 unit and may also have a difference of more than 2 units whereby good results are obtained. The calculation and the meaning of the solubility parameter are explained in "Properties of Polymers", their estimation and correlation with chemical structure by D.W. Krevelen Elsevier sc. Publ. Co. (1976), chapter 7, p. 129-159 and Polymer Handbook, 2nd edition, J. Brandrup and G.H. Immergut, chapter IV., p. 337-359, John Wiley Verlag (1975).

The set processing temperatures of these polymers are preferably within the range of 80°C to 190°C, preferably within the range of 95°C to 185°C.

The ratio of hydrophilic polymer to synthetic polymer can be 0.1:99.9 to 99.9:0.1. It is however preferred that the hydrophilic polymer contributes noticeably to the properties of the final material. Therefore, it is preferred that the hydrophilic polymer is present in an amount of at least 20%, more preferably 50% and most preferably in the range of 70% to 99% by weight of the entire composition, i.e. the synthetic polymer is present in a concentration of less than 80 %, more preferably less than 50%, and most preferably in a concentration in the range of 30% to 1% by weight of the entire composition.

A mixture of 2 to 15% by weight of the synthetic polymer and 98 to 85% of the hydrophilic polymer shows interesting variations of the properties of the obtained materials.

The synthetic polymer may contain the usual known additives.

The hydrophilic polymer may be formed into a melt in the presence of water and granulated before it is mixed with the synthetic polymer which is preferably granulated to an equal granular size as the hydrophilic polymer. However, it is possible to process the hydrophilic polymer together with powdered or granulated plastic material in any desired granule size, mixture or sequence.

The hydrophilic polymer/water component has a preferred water content in the range of about 10 to 20% by weight of the hydrophilic polymer/water component, preferably 12 to 19% and especially 14 to 18% by weight, calculated to the weight of the polymer/water component. This is especially true for gelatin. For other hydrophilic polymers the water content may be lower as is the case for several of the cellulose derivatives.

However, this is merely an experimental optimization and can easily be carried out by any expert in the art.

The water content herein refers to the weight of the hydrophilic polymer component within the total composition but is excluding the weight of the essentially water-insoluble synthetic thermoplastic polymer. It is essential that the hydrophilic polymer component has the indicated water content during melt formation.

As an "essentially water-insoluble synthetic thermoplastic polymer" a polymer is understood which preferably absorbs water up to a maximum 5% per 100 grams of the polymer at room temperature and preferably up to a maximum about 2% per 100 grams of the polymer at room temperature. Other polymers are considered as being water-swellable or water-soluble.

In order to form a melt of the new polymeric composition, the mixture is suitably heated in a screw and barrel of an extruder for a time long enough to effect melt formation. The temperature is preferably within the range of 80°C to 190°C, preferably within the range of 95°C to 185°C depending on the type of mixture used. For this, the composition is heated preferably in a closed volume. A closed volume can be a closed vessel or the volume created by the sealing action of the unmolten feed material as happens in the screw and barrel of injection moulding or extrusion equipment. In this sense the screw and barrel of an injection moulding machine or an extruder is to be understood as being a closed vessel. Pressures created in a closed vessel correspond to the vapour pressure of water at the used temperature but of course pressure may be applied and/or generated as normally occurs in a screw and barrel. The preferred applied and/or generated pressures are in the range of the pressures which occur in extrusion and are known per se, i.e. from zero to $150 \times 10^5$ N/m$^2$ preferably from zero to $75 \times 10^5$ N/m$^2$ and most particularly from zero to $50 \times 10^5$ N/m$^2$. The obtained melt can then be extruded and granulated.

However, the obtained melt in the screw and barrel may be injection molded directly into a suitable mold.

When forming a shaped article by extrusion the pressures are preferably as mentioned above. If the melt according to this invention is e.g. injection molded, the normal range of injection pressures used in injection moulding is applied, namely from $300 \times 10^5$ N/m$^2$ to $3.000 \times 10^5$ N/m$^2$ preferably $700 \times 10^5$ to $2200 \times 10^5$ N/m$^2$. The temperatures are the same as indicated above.

The polymer composition of the present invention may contain or may be mixed with additives such as fillers, lubricants, plasticizers and/or colouring agents.

These additives may be added before the melt-formation step or after this step, i.e. if the melt is first granulated, the additives may be added to the granulate before further processing.

Examples for fillers are inorganic fillers, such as the oxides of magnesium, aluminum, silicon, titanium, etc. preferably in a concentration in the range of about 0.02 to 3% by weight preferably 0.02 to 1% based on the total weight of all the components.

Examples for lubricants are stearates of aluminum, calcium, magnesium and tin as well as talc, silicones, etc. which may be present in concentrations of about 0.1 - 5 % preferably at 0.1 - 3 % based upon the weight of the total composition.

Examples of plasticizers include low molecular poly(alkylene oxides), such as poly(ethylene glycols), poly(propylene glycols), poly(ethylene-propylene glycols); organic plasticizers of low molar masses, such as glycerol, pentaerythritol, glycerol monoacetate, diacetate or triacetate; propylene glycol, sorbitol, sodium diethylsulfosuccinate, triethyl citrate, tributyl citrate, etc., added in concentrations ranging from 0.5 to 15%, preferably ranging from 0.5 to 5% based on the total weight of all the components. Examples of colouring agents include known azo dyes, organic or inorganic pigments, or colouring agents of natural origin. Inorganic pigments are preferred, such as the oxides of iron or titanium, these oxides, known per se, being added in concentrations ranging from 0.001 to 10 %, preferably 0.5 to 3 %, based on the weight of all the components.

The sum of the plasticizer and water contents within the hydrophilic component should preferably not exceed 20 %, and should most preferably not exceed 18 %, based on the weight of the hydrophilic component. For the different hydrophilic polymeric materials it is a simple experimental determination of the optimum water and plasticizer content which is no problem for a person skilled in the art.

There may further be added compounds to improve the flow properties of the starch material such as animal or vegetable fats, preferably in their hydrogenated form, especially those which are solid at room temperature. These fats have preferably a melting point of 50°C or higher. Preferred are triglycerides of $C_{12}$ -, $C_{14}$ -, $C_{16}$ -, and $C_{18}$ - fatty acids.

These fats can be added alone without adding extenders or plasticizers.

These fats can advantageously be added alone or together with mono- and/or diglycerides or phosphatides, especially lecithin. The mono- and diglycerides are preferably derived from the types of fats described above, i.e. from $C_{12}$ -, $C_{14}$ -, $C_{16}$ -, and $C_{18}$ - fatty acids.

The total amounts used of the fats, mono-, diglycerides and/or lecithins are up to 5% and preferably within the range of about 0.5 to 2% by weight of the hydrophilic component.

The materials described herein above form thermoplastic melts on heating and in a closed vessel, i.e. under conditions of controlled water-content and pressure. Such melts can be processed just like conventional thermoplastic materials, using, for example, injection molding, blow molding, extrusion and coextrusion (rod, pipe and film extrusion), compression molding, to produce known articles. The articles include bottles, sheets, films, packaging materials, pipes, rods, laminated films, sacks, bags, pharmaceutical capsules, granules or powders.

Such blended materials may be used as carrier materials for active substances, and may be mixed with active ingredients such as pharmaceuticals and/or agriculturally active compounds such as insecticides or pesticides for subsequent release applications of these ingredients. The resulting extruded materials can be granulated or worked to fine powders.

The following examples further explain the invention.

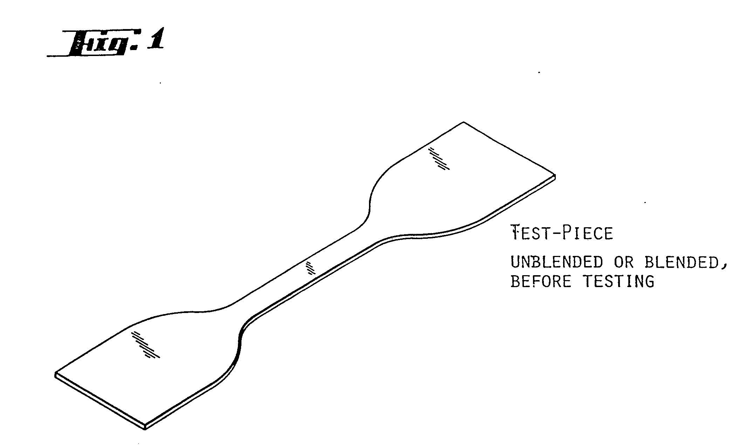

Figure 1 shows a test piece as produced for testing dimensional stability as described in Example 1.

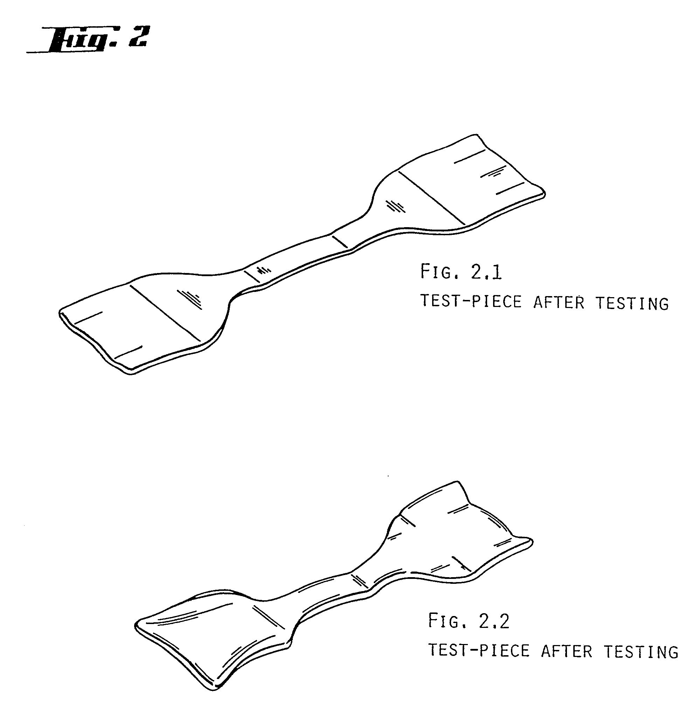

Figure 2 shows two test pieces of Figure 1 after it hade undergone dimensional testing as described in Example 1.

Example 1

Gelatin granules containing 17.0 % of water and 1 % of calcium stearate were mixed with a synthetic polymer in the weight ratios as specified in Table 1 below so that a freely flowing powder was obtained. This material was then fed to the hopper of an injection molding machine and injection molded to produce test pieces suitable for measuring their dimensional stability. The test pieces were injection molded using a standard injection molding machine with a temperature at the end of the barrel of between 155°C to 175°C and a cycle time of 20 seconds. The injection molding pressure varied between 800 bar and 1200 bar depending on the materials resp. blends used and the back pressure was 15 bar to 75 bar.

Testing and testing conditions

The test pieces were placed (laid on a screen) in a conditioning vessel in which a high relative humidity (near 100% R.H.) was maintained using a 1% aqueous sulfuric acid solution at room temperature. For each blends material 3 test pieces were used to obtain average figures relating to dimensional stability.

The test pieces obtained from the mold, were cut to a length of about 87-90 mm, which is close to the optimal length which can be measured on a NIKON profile projector V12.

After cutting, the samples were initially equilibrated to 14% water content, placed on the NIKON V12 and their widths and lengths were measured.

The samples were then placed in the conditioning vessel and exposed to a high relative humidity at room temperature. Reference samples of unblended gelatin were placed under same conditions. The dimensions were measured on each of the 3 pieces and recorded after 1, 2 and 5 days.

In width, slight expansions were generally observed.

According to this Example 1, the blends were prepared and tested as given in Table 1. The gelatin was an A-type gelatin of 240 Bloom.

TABLE 1

| NO | | SAMPLE MIXTURE | RATIO OF THE COMPONENTS | $T_e$ & $T_n$ & INJECTION MOLDING TEMP. | MELTING PRESSURE bar | INJECTION PRESSURE bar |
|---|---|---|---|---|---|---|
| | 1 | gelatin (references) | 100 % | 155°C | 15 | 1200 |
| | 2 | gelatin + polyethylene | 99 : 1 | 165°C | 15 | 1550 |
| | 3 | gelatin + polyethylene | 95 : 5 | 175°C | " | " |
| | 4 | gelatin + polystyrene | 95 : 5 | 175°C | " | 1500 |
| | 5 | gelatin + polystyrene | 90 : 10 | 155°C | " | 800 |
| | 6 | polyvinylether | 99 : 2 | 160°C | " | " |
| | 7 | gelatin + polyvinylether | 90 : 10 | 160°C | " | 1750 |
| | 8 | gelatin + polyvinylether | 75 : 25 | 160°C | " | " |
| | 9 | gelatin + POM | 95 : 5 | 155°C | " | 1000 |
| | 10 | gelatin + POM | 90 : 10 | " | " | 900 |
| | 11 | gelatin + EAA | 98 : 2 | " | " | 1100 |
| | 12 | gelatin + EAA | 95 : 5 | " | " | 1000 |
| | 13 | gelatin + EAA | 90 : 10 | " | " | 900 |

The dimensional stability was measured as described above and exemplary results to the shrinkage given in Table 2.

The shrinkage was measured in the longitudinal direction which was the direction of the injection molding. It is assumed that the pick up of water leads to a release of strain - the molecules being oriented initially in the direction of the injection molding -permitting the oriented molecules to revert to their unstrained de-oriented condition.

As compared with the unblended gelatin (reference) most of the blend samples show a strong reduction of the shrinkage in longitudinal direction. For instance for the 5% gelatin/POM blend only a slight shrinkage of about 1% during the first 5 days was observed.

Many of the test samples lost the added stability under the given conditions again after several days, e.g. after 10 days, which is a very useful property for a biodegradable material. Some of the blend additives have a stronger impact on the dimensional stability than others which is easily understood.

TABLE 2

| Example No. from Table 1 | after 1 day, % | after 2 days, % | after 5 days, % |
|---|---|---|---|
| 1 | - 7.3 | - 10.3 | - 10.3 |
| 5 | - 0.3 | + 1.0 | + 1.2 |
| 9 | - 0.8 | - 0.6 | - 1.2 |
| 10 | + 0.4 | + 0.3 | - 0.1 |
| 11 | - 5.0 | - 5.1 | - 6.1 |
| 12 | + 1.4 | + 2.2 | + 2.6 |
| 13 | + 1.1 | + 1.7 | + 2.0 |

Example 2

a) A batch of bone gelatin 200 bloom, grade B, in granulated form was conditioned as follows: The gelatin of which the water content was 10 % was filled into a drum and sprayed with a specially fine spray of water to the calculated content as wanted. The batch was then thoroughly mixed and stored in the closed drum for half a day at ambient temperature. The gelatin had a water content of 15.8 % and was extruded in an extruder and granulated to a granule size of about 2-3 mm diameter. The working conditions were:

| $T_b$ | $T_m$ | $T_e$ | $T_n$ |
|---|---|---|---|
| 125°C | 135°C | 140°C | 140°C |

$T_b$ = Temperature at the beginning of the screw.

$T_m$ = Temperature at the middle of the screw.

$T_e$ = Temperature at the end of the screw.

$T_n$ = Temperature at the nozzle.

The back pressure was 55 atm.

b) The granules obtained under a) were mixed with the components and in the ratio as described in Table 1 and injection molded into test pieces as described in Example 1. The results were analogous to those given in Table 2.

Example 3

92 parts of a bone gelatin, 150 bloom were mixed with 8 parts of a microfine cellulose and conditioned to a water content of 16.5 % by weight. There were added the synthetic polymers in the ratios and compositions as described in Table 1, Nos 10 to 14. The mixtures were used to produce test pieces on an injection molding machine with the set temperatures as indicated below:

$T_b$ = 135°C
$T_m$ = 145°C
$T_e$ = 160°C
$T_n$ = 160°C

The back pressure was 60 atm; the injection molding pressure 1400 atm.

Example 4

The following compositions of hydrophilic polymers were prepared:

a) - gelatin, bloom 150B, 60 parts;
- cellulose acetate phthalate, 26 parts;
- water, 14 parts

b) - hydroxypropyl methylcellulose-phthalate (HPMCP), 89 parts
- PE-glycol (10,000): 3.0 parts
- Ca-stearate: 3 parts
- water: 5 parts
    c) - gelatin, bloom 150B, 72 parts
- HPMCP, 10 parts
- water, 18 parts
    d) - gelatin, bloom 150B, 62 parts
- HPMC, 22.5 parts
- water, 15.5 parts
    e) - gelatin, bloom 150B, 60 parts
 - polyvinylpyrrolidone, 25 parts
- water, 15 parts
    f) - HPMCP, 57.4 parts
- glycerin, 4.1 parts
- PE-glycol (10,000), 1 part
- Ca-stearate, 2 parts
- cellulose, 27.6 parts
- water, 7.9 parts
    g) - HPMCP, 38.7 parts
- glycerin, 2.8 parts
- PE-glycol (10,000), 0.7 parts
- Ca-stearate, 1.3 parts
- polyvinylpyrrolidone, 43.5 parts
- water, 13 parts

These compositions were then thoroughly mixed with a synthetic polymer analogously to the synthetic polymers and the ratios of hydrophilic polymer to synthetic polymer as given in Example 1, Table 1, Nos 1 to 13 and injection molded to test pieces under the conditions as given in Example 1. Similar stability results were obtained.

## Claims

1. A polymer composition as obtained from a melt comprising at least one water-containing destructurized hydrophilic polymer and at least one synthetic essentially water-insoluble thermoplastic polymer.

2. A polymer composition according to claim 1, wherein the hydrophilic polymer is selected from the group consisting of animal gelatin, vegetable gelatins resp. proteins; acrylated proteins; water-soluble polysaccharides; alkylcelluloses, hydroxyalkylcelluloses and hydroxyalkylalkylcelluloses, carboxyalkylcellulose esters, water-soluble synthetic polymers, phthalated gelatin, gelatin succinate, crosslinked gelatin, shellac, cationically modified acrylates and methacrylates possessing, a tertiary or quaternary amino group.

3. A polymer composition according to the claims 1 or 2, wherein the thermoplastic polymer is selected from the group consisting of polyolefines, vinylpolymers, polyacrylonitriles, polycarbazols, essentially water-insoluble poly(acrylic acid) esters or poly(methacrylic acid) esters; polyacetals, polycondensates, polycarbonates, poly(alkylene terephthalates); polyarylethers; thermoplastic polyimides; poly(hydroxy butyrate), high molar-mass, essentially water-insoluble poly(alkylene oxides).

4. A polymer composition according to anyone of the claims 1 to 3, wherein the thermoplastic polymer is selected from the groups consisting of polyethylene, polyisobutylenes, polypropylenes; poly(vinyl chloride), poly(vinyl acetates), polystyrenes; polyamides, thermoplastic polyesters, polymers of ethylene oxide and propylene oxide and their copolymers; essentially water-insoluble thermoplastic copolymers preferably ethylene/vinyl acetate-copolymers, ethylene/vinyl alcohol-copolymers, ethylene/acrylic acid-copolymers, ethylene/ethyl acrylate-copolymers, ethylene/methyl acrylate-copolymers, ABS - copolymers, styrene/acrylonitrile-copolymers and mixtures thereof.

5. A polymer composition according to anyone of the claims 1 to 4, wherein the thermoplastic polymer has a set processing temperature preferably within thr range of 95°C to 210°C, preferably within the range of 95°C to 190°C.

6. A polymer composition according to anyone of the claims 1 to 5, wherein the thermoplastic essentially water-insoluble polymer contains polar groups preferably ether-, acid or ester groups.

7. A polymer composition according to anyone of the claims 1 to 6, wherein the ratio of hydrophilic polymer to synthetic polymer is 0.1:99 to 99.9:0.1 preferably that the hydrophilic polymer is present in an amount of at least 20%, more preferably 50% and most preferably in the range of 70% to 99% by weight of the entire composition.

8. A polymer composition according to anyone of the claims 1 to 7, wherein the hydrophilic polymer/water component has a water content in the range of about 10 to 20% by weight, preferably 12 to 19% and especially 14 to 18% by weight, calculated to the weight of the hydrophilic polymer/water component.

9. A polymer composition according to anyone of the claims 1 to 8, wherein the melt has been formed at a set temperature of about 80°C to about 190°C and a pressure at least equal to the vapour pressure of water at the used temperature.

10. A polymer composition according to anyone of the claims 1 to 9, wherein the polymer composition further contains fillers, lubricants, plasticizers and/or coloring agents.

11. A polymer composition according to anyone of the claims 1 to 10, wherein the inorganic filler or a mixture of such fillers is present in an amount of about 0.02 to 3 % by weight based on the weight of all components.

12. A polymer composition according to anyone of the claims 1 to 11, wherein the lubricant or a mixture of such lubricants is present in concentrations of about 0.1 to 3 % based upon the weight of the total composition.

13. A polymer composition according to anyone of the claims 1 to 12, wherein the plasticizer or a mixture of such plasticizers is present in an amount of about 0.5 to 15 %, preferably 0.5 to 5 % by weight of all components.

14. A composition according to anyone of the claims 1 to 13, wherein a coloring agent or a mixture of such coloring agents is present in concentrations ranging from 0.001 to 10 %, preferably 0.5 to 3 %, based on the weight of all the components.

15. A composition according to anyone of the claims 1 to 14, wherein the sum of the plasticizer and water does not exceed 20 %, and preferably does not exceed 18 %, based on the weight of all the components.

16. A composition according to anyone of the claims 1 to 15, wherein the polymer compostion contains at least one active ingredient preferably selected from pharmaceuticals and/or agriculturally active compounds.

17. A composition according to anyone of the claims 1 to 16 in the form of a melt.

18. A composition according to anyone of the claims 1 to 16 in the form of a solid shaped article.

19. A method of producing a polymer composition according to anyone of the claims 1 to 17 in form of a melt, comprising at least one modified destructurized hydrophilic polymer by heating a hydrophilic polymer having a water content of 5 to 25% by weight based on the total amount of hydrophilic polymer/water component and optionally containing further additives in a closed volume to elevated temperatures thereby at elevated pressures for a time long enough to form a melt, characterized in that said hydrophilic polymer water material is mixed with at least one synthetic thermoplastic polymer before melting is initiated.

20. A solid article as obtained from a melt produced according to claim 19.

21. The use of the polymer compositions as claimed in anyone of the claims 1 to 18 as carrier material for active ingredients, preferably as carrier materials for pharmaceuticals, and/or agriculturally active substances.

22. The process of shaping a polymer composition according to anyone of the claims 1 to 17, under controlled water content and pressure conditions as a thermoplastic melt wherein said shaping process is at least one member selected from the class consisting of injection molding, blow molding, extrusion and coextrusion, compression molding or vacuum forming.

23. The composition of claim 18, wherein said articles have been shaped as bottles, sheets, films, packaging materials, pipes, rods, laminates, sacks, bags or pharmaceutical capsules, granules or powders.

24. The use of an essentially water-insoluble thermoplastic polymer as a thermoplastic component in the thermoplastic processing of a water-containing hydrophilic polymer.

## Fig. 1

TEST-PIECE
UNBLENDED OR BLENDED,
BEFORE TESTING

*Fig. 2*

FIG. 2.1
TEST-PIECE AFTER TESTING

FIG. 2.2
TEST-PIECE AFTER TESTING